# FASCICULE DE BREVET EUROPEEN

(11) **EP 0 859 623 B1**
(45) Date de publication et mention de la délivrance du brevet: **13.03.2002**
(21) Numéro de dépôt: 96934887.9
(22) Date de dépôt: 10.10.1996
(51) Int. Cl.: A61K 35/78

(54) **COMPOSITION PROPHYLACTIQUE ABSORBABLE AYANT UN EFFET PROTECTEUR CONTRE LES ONDES ELECTROMAGNETIQUES A EFFET IONISANT OU NON IONISANT**
PROPHYLAKTISCHE ABSORBIERBARE ZUBEREITUNG MIT PROTEKTIVER WIRKUNG GEGEN ELEKTROMAGNETISCHE WELLEN MIT IONISIERENDEM ODER NICHT IONISIERENDEM EFFEKT
ABSORBABLE PROPHYLACTIC COMPOSITION FOR PROTECTION AGAINST IONISING OR NON-IONISING ELECTROMAGNETIC WAVES

(30) Priorité: 13.10.1995 FR 9512034; 08.10.1996 FR 9612247
(43) Date de publication de la demande: 26.08.1998
(73) Titulaire: Compagnie Generale de Dietetique, 14050 Caen (FR)
(72) Inventeur: LEGRAND, Charles, 14000 Caen (FR); BOUHAMIDI, Rachid, 14440 Douvres-La-Délivrande (FR)
(74) Mandataire: Cabinet HERRBURGER
(86) Numéro de dépôt international: FR9601577
(87) Numéro de publication internationale: WO9713521

(56) Documents cités:
- FR-A- 2 092 743
- FR-A- 2 372 823
- COMPTES RENDUS HEBDOMADAIRES DES SEANCES DE L'ACADEMIE D'AGRICULTURE DE FRANCE, vol. 58, no. 7, 1972, pages 452-460, XP000576292 MICHEL FLANZY ET AL.: "LA TENEUR ET LA R PARTITION DES DIVERS COMPOS S PH NOLIQUES DANS LE RAISIN ET LA RAFLE DE DOUZE C PAGES."
- DATABASE WPI Week 9229 Derwent Publications Ltd., London, GB; AN 92-239900 XP002008367 & JP,A,04 164 030 (YHUTOKU YAKUHIN KK) , 9 Juin 1992 cité dans la demande

## Description

La présente invention concerne l'utilisation de fractions résiduelles du raisin après foulage pour la préparation d'une composition prophylactique absorbable ayant un effet protecteur contre les ondes électromagnétiques à effet ionisant ou non ionisant.

La production de radicaux libres et, en particulier, de radicaux libres oxygénés est un processus physiologique normal au niveau cellulaire n'ayant, normalement, pas de conséquences délétères vu que l'organisme humain est pourvu de systèmes de protection : ces systèmes de protection sont constitués soit par des systèmes enzymatiques piégeurs de radicaux libres oxygénés tels la SOD ou susceptibles de décomposer des produits toxiques secondaires (H₂O₂) tels la catalase, soit par des systèmes non enzymatiques tels la vitamine E ou le glutathion.

Il arrive toutefois que le niveau de production des radicaux libres oxygénés excède les capacités des systèmes protecteurs et que ceux-ci réagissent avec d'autres molécules des cellules vivantes, ce qui peut avoir pour conséquence de profondes perturbations de l'intégrité membranaire, l'inactivation de nombreux systèmes enzymatiques et de profondes modifications de la structure des acides nucléiques.

Cette hyperproduction de radicaux libres oxygénés peut résulter :
- soit d'une activation de mécanismes endogènes connus comme générateurs de radicaux libres oxygénés tels le métabolisme énergétique au niveau des chaînes respiratoires ou l'activité des macrophages particulièrement stimulés en cas d'inflammation,
- soit d'une genèse induite par des facteurs exogènes tels que les ondes électromagnétiques (radiations ionisantes ou non ionisantes) ou une hyperoxygénation.

Parmi les mécanismes induits par une production excessive de radicaux libres oxygénés, les scientifiques s'accordent à considérer que le plus important est la lipoperoxydation qui correspond à la dégradation sous l'effet de réactions radicalaires des acides gras poly-insaturés présents dans les phospholipides constitutifs des membranes des cellules vivantes ; en conséquence, ces espèces moléculaires sont la cible privilégiée des attaques radicalaires.

On a pu déterminer expérimentalement que sous l'effet des radicaux libres oxygénés, les molécules d'acide gras poly-insaturés tendent à s'oxyder et à générer de nombreux produits de dégradation parmi lesquels les plus importants sont des aldéhydes, notamment le malondialdéhyde. Ce catabolisme a pour conséquence une perte de l'intégrité et de la stabilité des acides gras poly-insaturés, ce qui se traduit par une altération profonde de l'intégrité cellulaire.

Il est généralement admis que ces phénomènes sont impliqués dans des pathologies aussi variées que la polyarthrite rhumatoïde, certains cancers, certaines maladies neurodégénératives (Alzheimer, Parkinson), les ischémies cérébrales et cardiaques et, d'une façon générale, le vieillissement et la mort cellulaire.

A côté des systèmes de protection classiques susmentionnés (SOD, catalase, vitamine E, ...), il est connu depuis plusieurs années que certains flavonoides d'origine végétale sont d'excellents piégeurs de radicaux libres. On a pu constater que dans ce groupe, les plus intéressants semblent être les procyanidols ou les proanthocyanidines qui correspondent à des tanins hydrolysables constitués par une molécule glucidique sur laquelle est estérifié de l'acide gallique ou un de ses dérivés et qui sont capables de libérer du cyanidol par hydrolyse en milieu acide.

Suivant leur degré de polymérisation croissant, les procyanidols sont caractérisés par les termes suivants : monomères, dimères, trimères, tétramères, oligomères et condensés ; parmi les monomères, les plus courants sont l'épigallocatéchine et l'épigallocatéchine-gallate. On a en effet constaté que ces composés ont une très haute efficacité intrinsèque et sont en particulier nettement plus actifs que la vitamine E.

Le document FR 2 372 823 décrit un procédé d'obtention d'oligomers flavonoliques à partir notamment de pépins de raisins. De tels extraits sont, selon ce document, utilisables pour le traitement des affections du système circulatoire. Le document FR 2 092 743 concerne également un procédé d'obtention d'oligomères flavonoliques par extraction à partir de végétaux, tels que la vigne, le pommier etc ....

A partir de ces connaissances générales, on a déjà proposé, conformément à la publication JP-A-04/164 030, une composition prophylactique pour la protection contre les troubles liés à la radioactivité - tels qu'ils peuvent par exemple surgir chez des patients qui sont soignés par radiothérapie ou chez les manipulateurs permanents d'appareils émettant des rayonnements radioactifs - susceptible d'être absorbée par voie orale et dont les constituants actifs sont l'épigallocatéchine ou l'épigallocatéchine-gallate monomères préalablement purifiées. Cette publication japonaise indique que cette composition prophylactique peut être constituée par un mélange d'épigallocatéchine et d'épigallocatéchine-gallate dans le rapport 1/3 obtenu à partir de feuilles de thé vert.

La préparation de cette composition présente, toutefois, l'inconvénient de nécessiter des opérations d'extraction et de purification longues et coûteuses, donc particulièrement incommodes.

Conformément à l'invention, on a eu l'idée de remédier à ces inconvénients grâce à l'utilisation, pour la préparation d'une composition prophylactique absorbable ayant un effet protecteur contre les radiations ionisantes ou non ionisantes., non pas d'une composition à base de constituants préalablement isolés mais d'un extrait brut des fractions résiduelles du raisin après foulage, c'est-à-dire d'un produit disponible en grandes quantités et donc peu onéreux.

Selon l'invention, un tel extrait brut peut renfermer des pépins et/ou des rafles et/ou des pellicules. Il peut également être obtenu à partir de tourteaux résiduels provenant de la fabrication de l'huile de pépins de raisins.

Il est à noter qu'au cours des opérations de vinification, les grappes de raisin sont foulées, c'est-à-dire écrasées en partie afin de libérer le moût qui contient environ 80 % de jus et 20 % de matières solides essentiellement constituées par les pépins, les rafles et les pellicules. Ce moût est ensuite transféré dans une cuve de fermentation dans laquelle se produisent les différentes réactions chimiques de macération et de fermentation qui permettent la transformation du raisin en vin. Au cours de ces réactions, une partie des constituants initialement contenus dans les matières solides diffuse dans le jus. Les matières solides résiduelles sont ensuite séparées du jus puis pressées de manière à obtenir un vin dit « de presse » et un résidu nommé marc.

Ce résidu peut être distillé pour la production d'eau de vie, ou encore être utilisé en alimentation animale.

Un autre débouché des marcs de vinification plus ou moins séchés correspond à la fabrication de l'huile de pépins de raisins par une opération de pressage au cours de laquelle la partie grasse est séparée d'un tourteau.

Il est à noter que l'on a déjà proposé d'utiliser des sous-produits de la vinification pour la préparation après purification d'un médicament destiné au traitement des problèmes de circulation veineuse : l'Endotelon® .

Malgré les diverses possibilités susmentionnées, la majeure partie des marcs de vinification et des tourteaux provenant de la fabrication de l'huile de pépins de raisins est aujourd'hui mal rentabilisée.

Or, différentes études antérieures ont permis de démontrer que les pépins de raisins présents dans ces résidus, sont particulièrement riches en procyanidols et renferment, en particulier, d'importantes quantités d'épigallocatéchine et d'épigallocatéchine-gallate pouvant se trouver sous forme monomère mais également sous forme dimère, trimère, et oligomère.

Conformément à l'invention, on a vérifié que ces procyanidols sont également au moins partiellement présents dans les sous-produits de vinification susmentionnés, ce à côté de constituants autres tels la catéchine, ou encore l'acide gallique ou l'acide caféique.

On a, par suite, proposé d'utiliser un extrait brut de toutes ou parties des fractions résiduelles du raisin après foulage pour la préparation d'une composition prophylactique absorbable ayant une action protectrice contre les ondes électromagnétiques à effet ionisant ou non ionisant.

On a pu constater qu'un tel extrait brut a, de manière surprenante, une efficacité nettement supérieure à celle de l'épigallocatéchine et de l'épigallocatéchine-gallate isolées ou même associées entre elles sur la prévention des altérations lipidiques induites par les radiations.

Compte tenu de ce qui précède, l'utilisation conforme à l'invention présente l'avantage de permettre de valoriser les sous-produits des cépages vinicoles.

L'invention vise l'utilisation d'un tel extrait pour la fabrication d'une composition prophylactique absorbable ayant un effet protecteur contre les ondes électromagnétiques ionisantes ou non ionisantes.

Selon l'invention, cette composition prophylactique est caractérisée en ce qu'elle est constituée par un extrait brut de toutes ou parties des fractions résiduelles du raisin après foulage renfermant essentiellement de la catéchine, de l'épicatéchine et de l'acide gallique, la catéchine et l'épicatéchine pouvant soit être associées à l'acide gallique, soit être sous une forme non galloylée et ces composés se présentant sous forme de monomères ou sous forme d'oligomères homogènes ou hétérogènes ayant différents degrés de polymérisation, et en ce qu'elle renferme 10 à 30 % de monomères, 10 à 30 % de dimères, 10 à 30 % de trimères et 30 à 60 % de tétramères.

Selon une autre caractéristique de l'invention, la composition renferme des traces d'acide caféique.

A titre d'exemple, un tel extrait brut peut être obtenu par un procédé connu en lui même par lequel :
- on pulvérise une préparation à base de pépins de raisins,
- on soumet la poudre ainsi obtenue à une première extraction par de l'acétone,
- on sépare l'extrait ainsi obtenu par filtration,
- on lui ajoute du chlorure de sodium jusqu'à saturation,
- on évapore l'acétone,
- on reprend l'extrait par de l'eau distillée,
- on soumet la solution ainsi obtenue à une seconde extraction par de l'acétate d'éthyle,
- on sépare la phase organique et on soumet celle-ci à une lyophilisation de manière à obtenir l'extrait brut.

Il est à noter qu'il est avantageux d'effectuer la pulvérisation de la préparation de base en présence de neige carbonique pour la facilité et obtenir une meilleure homogénéité.

Il est en outre à noter que cette préparation de base est en règle générale relativement riche en composés apolaires qu'il convient d'éliminer pour obtenir en fin de processus une composition satisfaisante.

A cet effet, il est avantageux de soumettre la solution à une extraction intermédiaire par de l'éther diéthylique avant la mise en oeuvre de la seconde extraction.

Une préparation à base de pépins de raisins d'origine bordelaise a à titre d'exemple été traitée par le procédé susmentionné en utilisant de l'acétone à 70 % pour la première extraction.

Après lyophilisation, on a ainsi obtenu une composition renfermant 34 % de catéchine, 36 % d'épicatéchine, 11,6 % d'acide gallique et 0,007 % d'acide caféique.

On a en outre constaté que cette composition renfermait 19 % de monomères, 18 % de dimères, 21 % de trimères et 42 % de tétramères.

Compte tenu de sa bonne absorption intestinale et de sa diffusion rapide dans l'organisme, l'apport quotidien « per os » de l'extrait brut susmentionné représente une supplémentation nutritionnelle régulière permettant de lutter contre la production de radicaux libres oxygénés induite sous l'action de rayonnements ou d'un stress oxydatif dépassant les capacités endogènes de protection.

En ce qui concerne les recommandations d'emploi, la quantité d'extrait à ingérer peut varier selon les cas, mais il est en règle générale souhaitable d'utiliser chez l'homme adulte des doses quotidiennes de 20 mg à 3 g.

La limite inférieure de cette fourchette s'applique à titre d'exemple aux personnes régulièrement exposées à de faibles radiations, tandis que la limite supérieure est plus spécialement adaptée à des personnes soumises à une irradiation intense, mais de courte durée.

Différents tests ont permis de démontrer l'efficacité de l'extrait brut conforme à l'invention contre les altérations induites par les radiations ionisantes (rayons X) et les radiations non ionisantes (UV-C). L'efficacité de cet extrait a d'abord été étudiée sur les acides gras poly-insaturés purs en solution aqueuse après induction du stress oxydatif par les rayons UV-C (200 µW/cm² ; 24 h) . On a ainsi pu obtenir une protection totale de ces molécules à partir de 2 mg/l d'extrait dans le milieu.

On a ensuite étudié l'effet de cet extrait sur les atteintes induites par les rayons UV-C sur les lipides membranaires du cerveau et du foie de souris. On a, là encore, apporté la preuve de l'efficacité totale de cet extrait à partir d'une concentration de 4 mg/l.

Dans une troisième étape, on a étudié l'effet des radiations UV-C et X sur des cellules fibroblastiques humaines en culture ainsi que la protection apportée par l'extrait brut conforme à l'invention. Le suivi de la lyse cellulaire après irradiation UV-C (200 µW/cm² ; 1 h) a montré que la protection exercée par cet extrait sur les cellules en culture était dose-dépendante et qu'elle est supérieure à 50 % pour une concentration en extrait de 40 mg/l.

Pour les rayons X, la viabilité cellulaire a été prise en considération. Cet index a été évalué par dénombrement cellulaire mais également par suivi du pouvoir de prolifération des cellules remises en culture après irradiation aux rayons X (30 Grays à raison de 2 Gy/min). Ceci a permis de constater que, à partir de 20 mg/l d'extrait brut dans le milieu, le nombre de cellules mortes est significativement réduit. En outre, la prolifération-des cellules qui est bloquée chez les cellules irradiées en l'absence d'extrait est en grande partie restaurée chez les cellules irradiées en présence de cet extrait.

On a, enfin, étudié l'effet de l'extrait conforme à l'invention sur le système hématopoiétique après irradiation totale de rats mâles avec des rayons X (5 Gy en 3 séances). Auparavant, une série de rats ont été gavés avec 60 mg/jour d'extrait. Six jours après la dernière irradiation, les animaux ont été anesthésiés et le sang a été prélevé au niveau de la carotide. Une analyse sanguine a été réalisée sur chacun des échantillons.

Avant irradiation, aucun effet toxique n'a été observé chez les animaux ayant ingéré la préparation conforme à l'invention.

Les résultats de cette expérience ont montré qu'il y avait une diminution de tous les constituants et paramètres sanguins (hématies, plaquettes, hématocrites, hémoglobine, polyneutrophiles, lymphocytes et monocytes) chez les rats irradiés sans prétraitement et qu'il y avait une nette amélioration chez les animaux préalablement gavés avec l'extrait brut conforme à l'invention.

A titre d'exemple on a effectué un dénombrement plaquettaire chez des animaux témoins, des animaux irradiés et des animaux irradiés préalablement traités avec l'extrait conforme à l'invention. On a ainsi obtenu les résultats suivants :
- animaux témoins 327,5 ± 103,5 G/l
- animaux irradiés 152,66 ± 49,93 G/l
- animaux traités puis irradiés 277,66 ± 78,54 G/l

Ces résultats montrent clairement l'efficacité in vivo de l'extrait conforme à l'invention.

Pour mettre en lumière la supériorité de l'extrait brut conforme à l'invention par rapport à l'épigallocatéchine et l'épigallocatéchine-gallate isolées, conformément à la publication JP-A-04/164 030, on a irradié par des UV-C (200 µW/cm²) durant 15 h une préparation de microsomes de foie de babouins en présence d'épigallocatéchine (EGC), d'épigallocatéchine-gallate (EGCG) ou de l'extrait brut conforme à l'invention (I).

Les additions ont été effectuées de façon à avoir toujours la même quantité d'épicatéchine, à savoir 1,45 mg/l.

On a ensuite procédé à la quantification des acides gras poly-insaturés résiduels (AGPI) (20:4 n-6 et 22:6 n-3) ainsi que du malondialdehyde (MDA) qui est un produit de leur dégradation.

Ce test a permis d'aboutir aux résultats suivants :

| **Concentration** | **MDA (µM)** | **AGPI** | |
|---|---|---|---|
| | | 20:4 n-6(µg/ml) | 22:6 n-3 (µg/ml) |
| Témoins non irradiés | 0 | 137.85 ± 2.65 | 42.43 ± 14.0 |
| Echantillons irradiés sans additif | 34.17 ± 4.07 | 081.73 ± 7.65 | 15.40 ± 1.57 |
| Echantillons irradiés + I | 22.66 ± 1.75 | 136.29 ± 0.08 | 45.25 ± 9.61 |
| Echantillons irradiés + EGC | 32.02 ± 2.60 | 094.53 ± 9.11 | 24.05 ± 2.36 |
| Echantillons irradiés + EGCG | 32.25 ± 0.76 | 098.59 ± 21 | 31.40 ± 1.48 |

Ces résultats prouvent que l'irradiation des microsomes entraîne une dégradation des acides gras poly-insaturés et une augmentation de la production de malondialdéhyde.

L'extrait conforme à l'invention semble apporter une protection significative des microsomes contre ces altérations ce qui n'est pas le cas de l'épigallocatéchine et de l'épigallocatéchine-gallate isolées.

On a ensuite repris ce même test en irradiant une préparation de microsomes de foie de babouin à plus faible concentration protéique en présence de l'extrait brut de pépins de raisins conforme à l'invention et d'un mélange constitué de 1/4 d'épigallocatéchine et de 3/4 d'épigallocatéchine-gallate.

Les additions ont là encore été réalisées de façon à avoir 1,45 mg/l d'épicatéchine dans tous les cas.

Les analyses effectuées similaires au test susmentionné ont permis d'aboutir aux résultats suivants :

| **Concentration** | **MDA (µM)** | **AGPI** | |
|---|---|---|---|
| | | 20:4 n-6(µg/ml) | 22:6 n-3 (µg/ml) |
| Témoins non irradiés | 0.378 ± 0.05 | 18.96 ± 0.86 | 8.21 ± 1.85 |
| Echantillons irradiés sans additif | 14.93 ± 0.35 | 6.66 ± 0.11 | indétectable |
| Echantillons irradiés + I | 5.91 ± 1.76 | 16.59 ± 0.01 | 7.51 ± 0.47 |
| Echantillons irradiés + mélange EGC/EGCG | 14.186 ± 0.37 | 09.59 ± 1.15 | 2.95 ± 0.99 |

Ce test a permis encore une fois de prouver que l'extrait conforme à l'invention permet une protection des microsomes contre les altérations induites par les rayons UV-C alors que le mélange épigallocatéchine/épigallocatéchine-gallate semble totalement inefficace aux doses utilisées.

## Revendications

1. Utilisation d'un extrait brut des fractions résiduelles du raisin après foulage pour l'obtention d'une composition prophylactique absorbable destinée à la protection contre les ondes électromagnétiques à effet ionisant ou non ionisant.

2. Utilisation selon la revendication 1,
**caractérisée en ce que**
l'extrait brut renferme essentiellement de la catéchine, de l'épicatéchine et de l'acide gallique, la catéchine et l'épicatéchine pouvant soit être associées à l'acide gallique, soit être sous une forme non galloylée et ces composés se présentant sous forme de monomères ou sous forme d'oligomères homogènes ou hétérogènes ayant différents degrés de polymérisation, et **en ce que** l'extrait brut renferme 10 à 30 % de monomères, 10 à 30 % de dimères, 10 à 30 % de trimères et 30 à 60 % de tétramères.

3. Utilisation selon la revendication 2,
**caractérisée en ce que**
l'extrait brut renferme des traces d'acide caféique.

## Claims

1. Use of a crude extract of the residual fractions of grapes after pressing for obtaining an absorbable prophylactic composition for protection against electromagnetic waves having an ionising or non-ionising effect.

2. Use according to claim 1, **characterised in that** the crude extract basically comprises catechin, epicatechin and gallic acid, it being possible for the catechin and the epicatechin either to be associated with the gallic acid or to be in a form not associated with the gallic acid and those compounds being in the form of monomers or in the form of homogeneous or heterogeneous oligomers having different degrees of polymerisation, and **in that** the crude extract contains from 10 to 30% of monomers, from 10 to 30% of dimers, from 10 to 30% of trimers and from 30 to 60% of tetramers.

3. Use according to claim 2, **characterised in that** the crude extract contains traces of caffeic acid.

## Patentansprüche

1. Verwendung eines Rohextrakts aus Restfraktionen von Weintrauben nach dem Keltern zur Gewinnung einer absorbierbaren prophylaktischen Zusammensetzung zum Schutz vor elektromagnetischer Strahlung mit oder ohne ionisierende Wirkung.

2. Verwendung nach Anspruch 1, **dadurch gekennzeichnet, dass** der Rohextrakt im wesentlichen Katechin, Epikatechin und Gallussäure enthält, wobei das Katechin und das Epikatechin in mit der Gallussäure assoziiert oder in Nicht-Gallat-Form vorliegen können und diese Verbindungen in Form von Monomeren oder von homogenen oder heterogenen Oligomeren mit unterschiedlichen Polymerisationsgraden vorliegen, und dass der Rohextrakt 10 bis 30% Monomere, 10 bis 30% Dimere, 10 bis 30% Trimere und 30 bis 60% Tetramere enthält.

3. Verwendung nach Anspruch 2, **dadurch gekennzeichnet, dass** der Rohextrakt Spuren von Koffeinsäure enthält.
